# EUROPÄISCHE PATENTSCHRIFT

(11) **EP 2 593 767 B1**
(45) Veröffentlichungstag und Bekanntmachung des Hinweises auf die Patenterteilung: **02.01.2019**
(21) Anmeldenummer: 11779329.9
(22) Anmeldetag: 17.06.2011
(51) Int. Cl.: G01N 1/22, G01N 1/40, G01N 1/02

(54) **VERFAHREN UND VORRICHTUNG ZUR DETEKTION VON SPRENGSTOFFPARTIKELN IN EINEM GASSTROM**
METHOD AND DEVICE FOR DETECTING EXPLOSIVE-SUBSTANCE PARTICLES IN A GAS FLOW
PROCÉDÉ ET DISPOSITIF DE DÉTECTION DE PARTICULES D'EXPLOSIF DANS UN FLUX GAZEUX

(30) Priorität: 13.07.2010 DE 102010027074
(43) Veröffentlichungstag der Anmeldung: 22.05.2013
(73) Patentinhaber: Spherea GmbH, 89077 Ulm (DE)
(72) Erfinder: BEER, Sebastian, 93047 Regensburg (DE); ZIEMANN, Thomas, 84416 Inning am Holz (DE); FRIEDBERGER, Alois, 85667 Oberpframmern (DE)
(74) Vertreter: Mazabraud, Xavier
(86) Internationale Anmeldenummer: PCT/DE2011/001309
(87) Internationale Veröffentlichungsnummer: WO 2012/010123

(56) Entgegenhaltungen:
- WO-A1-2010/060899
- US-A- 5 854 431
- US-A1- 2003 192 363
- US-A1- 2006 272 393
- US-A1- 2010 130 796
- US-B1- 6 604 406
- VOICOLESCU I ET AL: "Micropreconcentrator for enhanced trace detection of explosives and chemical agents", IEEE SENSORS JOURNAL, IEEE SERVICE CENTER, NEW YORK, NY, US, Bd. 6, Nr. 5, 1. Oktober 2006 (2006-10-01) , Seiten 1094-1103, XP002493890, ISSN: 1530-437X, DOI: 10.1109/JSEN.2006.881431
- HANNUM D W ET AL: "Miniaturized explosives preconcentrators for use in man-portable explosives detection systems", SECURITY TECHNOLOGY, 2000. PROCEEDINGS. IEEE 34TH ANNUAL 2000 INTERNAT IONAL CARNAHAN CONFERENCE ON OCTOBER 23-25, 2000, PISCATAWAY, NJ, USA,IEEE, 23. Oktober 2000 (2000-10-23), Seiten 222-227, XP010527867, ISBN: 978-0-7803-5965-9

## Beschreibung

Die Erfindung betrifft ein Verfahren und eine Vorrichtung zur Detektion von Sprengstoffpartikeln in einem Gasstrom, bei dem der Gasstrom für einen vorgegebenen Zeitraum durch ein Adsorptionsnetz geleitet wird, so dass Sprengstoffpartikel darauf adsorbiert werden, anschließend das Adsorptionsnetz auf eine Heiztemperatur gewärmt wird, bei der die Sprengstoffpartikel desorbieren und ein Gasstrom mit den desorbierten Sprengstoffpartikeln einem Detektor zu deren Detektion zugeführt wird, wobei als Adsorptionsnetz ein Mikrofilter mit einer Porengröße kleiner als der Partikelgröße der zu detektierenden Sprengstoffpartikel verwendet wird.

Ein Detektionsverfahren bzw. eine Detektionsvorrichtung ist aus der US 6 604 406 B1 bekannt. US 2010/130796 A1 offenbart ein Detektionsverfahren, bei dem Hohlräume definiert sind, die mit einer gleichmäßigen Schicht aus PIM-Material bedeckt sind. Die Partikel passieren nicht durch das Material. Der zunehmende Einsatz von Sprengstoffen für terroristische Zwecke, insbesondere im zivilen Luftverkehr, erzeugt einen dringenden Bedarf für effiziente Sprengstoffdetektoren, wobei insbesondere mobile bzw. feldtaugliche Systeme notwendig sind. Wenn beispielsweise ein potentieller Terrorist einen Sprengstoff bearbeitet, hinterlässt dies geringe Sprengstoffspuren an Kleidung und Haut. Ziel eines Detektionsverfahrens für Sprengstoffspuren ist es, diese Sprengstoffspuren festzustellen, beispielsweise vor dem Betreten eines Flugzeugs. Dabei wird ein Gasstrom - meist Umgebungsluft - über einen zu untersuchenden Gegenstand bzw. eine zu untersuchende Person geleitet, wobei Sprengstoffpartikel mitgerissen werden, sofern solche vorhanden sind. Diese Art der Detektion ist jedoch erschwert durch die sehr niedrigen Konzentrationen der Sprengstoffe, die oftmals im Bereich von ppt (parts per trillion) liegen, wobei die direkte Detektion der Sprengstoffe in der Gasphase teilweise sehr schwer ist, da die Gleichgewicht-Gaskonzentrationen der üblichen Sprengstoffe sehr gering sind.

Ein Detektionsverfahren für Sprengstoffe ist in der US 6 604 406 beschrieben, bei der die zu suchenden Stoffe in Partikelform auf einem als Filz, Vlies oder Geflecht ausgebildeten Adsorptionsnetz gesammelt und anschließend einem Detektor zugeführt werden. Bei diesem vorbekannten Verfahren wird das Sprengstoffpartikel in niedriger Konzentration enthaltene Gas in einem ersten Adsorptionsschritt durch das als Filz, Vlies oder Geflecht ausgebildete Adsorptionsnetz gesaugt, wobei ein Teil der Partikel auf dem Filter adsorbieren und somit die Konzentration der Partikel auf dem Adsorptionsnetz mit der Zeit zunimmt. In einem zweiten Verfahrensschritt, dem Desorptionsschritt, wird das Adsorptionsnetz erhitzt und die Strömungsrichtung des Gasstromes durch das Adsorptionsnetz wird umgekehrt. Dabei desorbieren die angereicherten Sprengstoffpartikel vom Adsorptionsnetz und können in erhöhter Konzentration vom Detektor erfasst werden. Nachteilig dabei ist, dass nur größere Partikel im Absorptionsnetz hängen bleiben während die kleineren Partikel passieren und damit zur Detektion nicht beitragen können.

Ein eingangs genanntes Verfahren und die entsprechende Vorrichtung sind aus US 5 854 431 A bekannt.

Hiervon ausgehend liegt der Erfindung die Aufgabe zugrunde, die Detektierbarkeit der Sprengstoffe weiter zu verbessern bzw. die Detektionsgrenze weiter zu reduzieren.

Erfindungsgemäß wird diese Aufgabe dadurch gelöst, dass eine Heiztemperatur eingestellt und ein Mikrofilter mit einer Porengröße verwendet wird und dass die zu detektierenden Sprengstoffpartikel, nach dem Erwärmen und Desorbieren in der Gasphase, den Mikrofilter durchströmen. Hierbei wird unter dem Begriff "Mikrofilter" eine Membran mit einer Dicke im Bereich von ca. 1 µm verstanden, die durch Stützstrukturen mechanische Stabilität aufweist und regelmäßige Perforationen aufweist. Diese Perforationen weisen vorzugsweise identischen Durchmesser auf, der vorzugsweise kleiner 1µm, noch bevorzugter kleiner 400 nm, ist. Dies ermöglicht im Gegensatz zum Stand der Technik, dass alle im Gasstrom befindlichen Partikel aufgefangen bzw. auf diesem adsorbiert werden, während bei den herkömmlichen Systemen ein signifikanter Teil der Partikel durch das Geflecht des Adsorptionsnetzes gelangen kann, so dass die Anreicherung wesentlich schwächer ist bzw. länger dauert. Mittels des Mikrofilters können die Partikel auf der Oberfläche zurückgehalten werden, wodurch diese sehr leicht zugänglich bleiben und einfach wieder desorbiert werden können. Im Gegensatz dazu sind die herkömmlicherweise verwendeten Geflechte dreidimensionale Gewebe oder Filze. Diese erfindungsgemäße Ausbildung hat insbesondere im Desorbtionsschritt einen Vorteil, weil sich alle Partikel auf einer einzigen Oberfläche befinden und nicht in einer dreidimensionalen Struktur und somit eine gezielte Desorbtion durch die Erhitzung der Mikrofilteroberfläche möglich ist. Diese gezielte Erhitzung auf vorgegebene Temperaturen kann ferner dafür genutzt werden, durch die Einstellung gewisser Temperaturen eine Detektionsselektivität für bestimmte Sprengstoffe zu erreichen.

Damit kann vorteilhafterweise eine mobile Partikel-Gas-Koversion von kleinen Sprengstoffpartikeln ermöglicht werden. Die geringe thermische Masse des Mikrofilters erlaubt einen Betrieb mit niedriger Leistung und einen sehr schnellen Temperaturanstieg beim Erhitzungsvorgang. Damit ließe sich anstelle nur einer Desorption der Partikel auch eine Dissoziation erreichen, wobei Molekülgruppen abgespalten werden, was alternative Detektionsmöglichkeiten erlauben würde, z.B. den Nachweis von Molekülen mit Nitrogruppen.

Die Porengröße des Mikrofilters wird vorzugsweise in Abhängigkeit der zu detektierenden Sprengstoffe gewählt werden, so dass es auch möglich ist, Mikrofilter mit unterschiedlichen Porengrößen zur Detektion bestimmter Sprengstoffe zu verwenden. Es ist auch möglich, den Mikrofilter zu diesem Zweck austauschbar zu gestalten.

Um die Selektivität der Detektion weiter zu erhöhen, ist es auch möglich, zwei Mikrofilter mit unterschiedlichen Porendurchmessern hintereinander anzuordnen, wobei der erste Mikrofilter eine größere Porengröße (beispielsweise 1 µm) aufweist, um große unerwünschte Partikel abzufangen und stromab ein zweiter Mikrofilter mit einer geringeren Porengröße (beispielsweise 400 nm) vorgesehen ist, auf dem die zu detektierenden Partikel adsorbieren. Beim zweiten Verfahrensschritt wird nur der zweite Mikrofilter erwärmt, so dass nur die darauf adsorbierten Sprengstoffpartikel desorbieren und dem Detektor zugeführt werden. Dann könnte nach Beendigung des Detektionsprozesses zur Entfernung der am ersten Filter adsorbierten unerwünschten Partikel auch dieser Filter beheizt werden.

Gemäß der Erfindung wird eine Heiztemperatur eingestellt und ein Mikrofilter mit einer Porengröße verwendet, bei dem die zu detektierende Sprengstoffpartikel nach dem Erwärmen und Desorbieren in der Gasphase den Mikrofilter passieren können. Diese Temperatur liegt bei etwa 150° bis 250°. Bei dieser besonders einfachen Verfahrensausführung, die zudem eine baulich einfache Vorrichtung nutzen kann, ist keine Durchströmung der Anordnung mit unterschiedlichen Strömungsrichtungen nötig. Dabei wird der Gasstrom vorzugsweise permanent aktiviert, wobei der Mikrofilter permanent durchströmt und der Gasdetektor ständig vom Gasstrom überströmt wird. Aber erst nach einiger Zeit (insbesondere etwa 10-20 sek.), wenn genügend Partikel auf dem Mikrofilter adsorbiert sind, und der Mikrofilter erhitzt wird, ergibt sich bei der damit einhergehenden Desorption der angereicherten Sprengstoffpartikel eine hinreichende Konzentration derselben, die vom Detektor gut erfasst bzw. detektiert werden kann.

Eine bevorzugte Vorrichtung zur Durchführung des oben genannten Verfahrens umfasst einen Mikrofilter, hinter dem ein Detektor angeordnet ist, wobei der Mikrofilter eine Heizvorrichtung sowie eine Regelvorrichtung zur Regelung der Temperatur des Mikrofilters umfasst. Bei dieser einfachen Anordnung werden der Mikrofilter und der Detektor immer in der gleichen Richtung vom Gasstrom mit den Sprengstoffpartikeln durchströmt, was baulich sehr einfach ist.

Eine Vorrichtung zur Durchführung dieser Ausgestaltung des Verfahrens umfasst einen Strömungskanal mit einem Mikrofilter sowie einen Zirkulationskanal mit einem Detektor, der im Sammelmodus versperrbar und im Detektionsmodus mit dem Strömungskanal zur Bildung eines geschlossenen Ringkanals verbindbar ist.

Gemäß einer vorteilhaften Weiterbildung umfasst die Vorrichtung einen Halogenstrahler zur Erwärmung des Mikrofilters, wodurch entweder durch Verwendung eines Kollimators eine parallele gleichmäßige Bestrahlung des gesamten Mikrofilters oder durch Fokussierlinse eine gezielte Ausrichtung auf bestimmte Bereiche des Filters möglich ist.

In Verbindung mit einem optischen oder resistiven Thermometer kann die Temperatur des Mikrofilters genau gemessen werden, wodurch eine gezielte Einstellung einer bestimmten Temperatur möglich ist. Dies ermöglicht die Einstellung bestimmter vorgegebener zeitlicher Temperaturverläufe, wodurch eine Selektivität für unterschiedliche Sprengstoffarten erzielbar ist.

Ein Verfahren zur Herstellung eines Mikrofilters zur Verwendung einer der vorgeschriebenen Vorrichtungen wird vorzugsweise mittels eines photolithographischen Ätzverfahrens hergestellt. wodurch alle Poren des Mikrofilters mit identischem Durchmesser im gewünschten Größenbereich ausgeführt werden können.

Die Erfindung wird nachfolgend anhand bevorzugter Beispiele unter Bezugnahme auf die beigefügten Zeichnungen weiter erläutert. Gleiche Bezugszeichen bezeichnen in den verschiedenen Darstellungen gleiche Bauteile. Dabei zeigt:
- Figur 1:: eine erste Ausführungsform der Vorrichtung zur Detektion van Sprengstoffpartikeln;
- Figur 2:: eine zweite Ausführungsform, die nicht zur Erfindung gehört, der Vorrichtung zur Detektion von Sprengstoffpartikeln in zwei unterschiedlichen Betriebszuständen;
- Figur 3:: eine dritte Ausführungsform der Vorrichtung zur Detektion von Sprengstoffpartikeln.

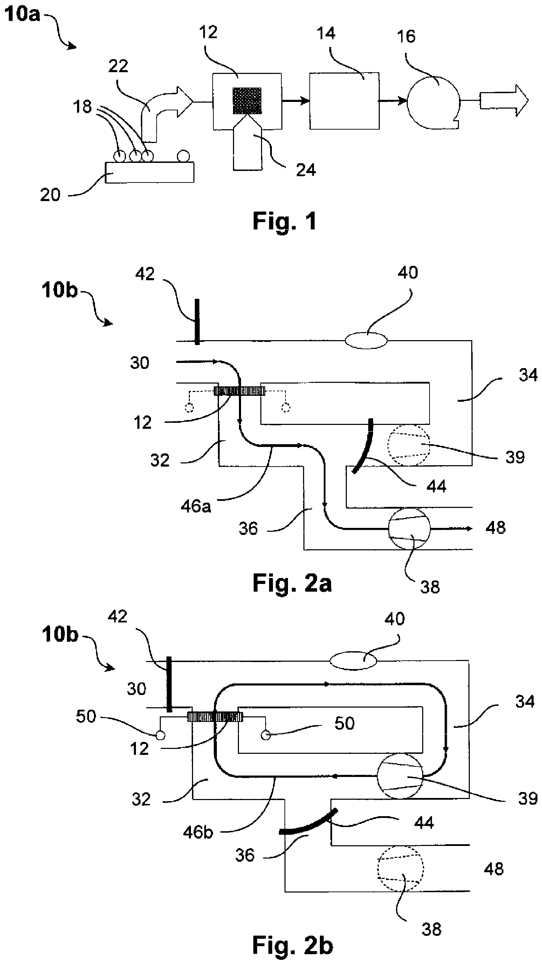

In **Figur 1** ist eine Ausführungsform einer Detektionsvorrichtung **10a** schematisch dargestellt, die im Wesentlichen aus einem Mikrofilter **12,** einem Detektor **14** und einer Saugpumpe **16** besteht. Schematisch ist ferner ein mit Sprengstoffpartikel 18 kontaminierter Gegenstand **20** dargestellt, über den ein Luftstrom **22** geleitet wird, der durch den Mikrofilter **12** strömt und weiter den Detektor **14** passiert. Dabei haften die in der Zeichnung stark vergrößert dargestellten Sprengstoffpartikel 18 am Mikrofilter 12 an, da diese aufgrund der gewählten Porengröße, die kleiner ist als die Größe der Sprengstoffpartikel **18**, den Mikrofilter **12** nicht passieren können. Nach einer gewissen Zeit, vorzugsweise ca. 10 bis 20 sek., haben sich genügend Sprengstoffpartikel **18** auf dem Mikrofilter **12** angereichert, so dass der Mikrofilter **12** mittels einer Heizvorrichtung **24** erwärmt wird, vorzugsweise auf eine Temperatur von etwa 150 bis 250°C. Durch die erhöhte Temperatur desorbieren die Sprengstoffpartikel **18** vom Mikrofilter **12** und gehen über in die Gasphase, in welcher dieser die Poren des Mikrofilters **12** passieren können und somit in erhöhter Konzentration dem Detektor **14** zuführbar sind. Nach einem gewissen Zeitraum von wenigen Sekunden, innerhalb dessen im Wesentlichen alle am Mikrofilter **12** anhaftenden Sprengstoffpartikel **18** desorbiert sind, wird die Heizvorrichtung **24** wieder abgeschaltet und es kann erneut mittels eines Gasstromes **22** ein weiterer zu untersuchender Gegenstand **20** auf Sprengstoffpartikel **18** untersucht werden.

In den **Figuren 2a** und **2b** ist ein Vergleichsbeispiel, das nicht zur Erfindung gehört, einer Vorrichtung **10b** zur Detektion von Sprengstoffpartikeln schematisch dargestellt. Diese umfasst einen Gaseinlass **30**, dem sich ein Strömungskanal 32 anschließt, in dem ein Mikrofilter **12** angeordnet ist. Der Strömungskanal **32** ist einerseits mit einem U-förmigen Zirkulationskanal **34** verbunden, der beiderseits des Mikrofilters 12 mit dem Strömungskanal 32 in Verbindung steht. Ferner ist der Strömungskanal 32 mit einem Auslasskanal 36 verbunden, in dem eine Saugpumpe 38 angeordnet ist. Im Zirkulationskanal **34** ist eine Zirkulationspumpe 39 angeordnet. In der Wand des Zirkulationskanals **34** ist ferner ein Detektor **40** angeordnet, bei dem es sich vorzugsweise um einen lonen-Mobilitäts-Spektrometer (IMS) oder einen Metalloxid-Halbleitergassenor (MOX-Sensor) handelt. Der Strömungskanal 32 ist gegenüber dem Einlass 30 durch einen Einlassverschluss **42** und gegenüber dem Auslasskanal 36 durch einen Auslassverschluss **44** absperrbar.

Die Vorrichtung **10b** ist in Figur 2a im Sammelmodus und in Figur 2b im Detektionsmodus dargestellt. Im Sammelmodus gemäß **Figur 2a** ist der Einlassverschluss **42** offen, so dass der Einlass **30** mit dem Strömungskanal 32 kommuniziert. Der Auslassverschluss **44**, der wechselweise entweder den Auslasskanal 36 oder den Zirkulationskanal 34 verschließt, befindet sich in der den Zirkulationskanal **34** verschließenden Stellung. Durch Betrieb der Saugpumpe 38 wird ein Gasstrom **46a** (vorzugsweise ein Umgebungsluftstrom) in den Einlass **30** gesaugt, von dort durch den Mikrofilter **12**, den Strömungskanal 32 und den Auslasskanal **36** geleitet und bis zu einem Gasauslass 48 geführt. Dabei bleiben die mit dem Gasstrom **46a** transportierten Sprengstoffpartikel aufgrund der geringeren Porengröße des Mikrofilters 12 an diesem hängen und aggregieren dort. Da der Auslassverschluss **44** den Zirkulationskanal **34** verschließt, wird dieser nicht durchströmt.

Nach einem Zeitraum von einigen Sekunden, wenn genügend Sprengstoffpartikel auf dem Mikrofilter 12 aggregiert sind, wird in den in **Figur 2b** dargestellten Detektionsmodus umgeschaltet, bei dem der Einlassverschluss **42** verschlossen ist und der Auslassverschluss **44** in die den Auslasskanal **36** verschließende Stellung umgelegt wird. Ferner wird die Saugpumpe 38 abgeschaltet und stattdessen die Zirkulationspumpe **39** aktiviert. In diesem Fall liegt ein geschlossener ringförmiger Strömungskanal vor, in dem der Gasstrom **46b** zirkuliert. Gleichzeitig wird über Kontakte **50** elektrischer Strom durch den Mikrofilter **12** geleitet, so dass sich dieser auf eine Temperatur erwärmt, bei der die Sprengstoffpartikel vom Mikrofilter 12 desorbieren. Während der Gasstrom **46b** zirkuliert, werden die am Mikrofilter **12** anhaftenden Sprengstoffpartikel desorbiert und passieren den Detektor **40** und werden dort erfasst. Die Zirkulationspumpe 39 wird so betrieben, dass der zirkulierende Gasstrom **46b** in entgegengesetzter Richtung wie der Gasstrom **46a** im Sammelmodus den Mikrofilter **12** passiert.

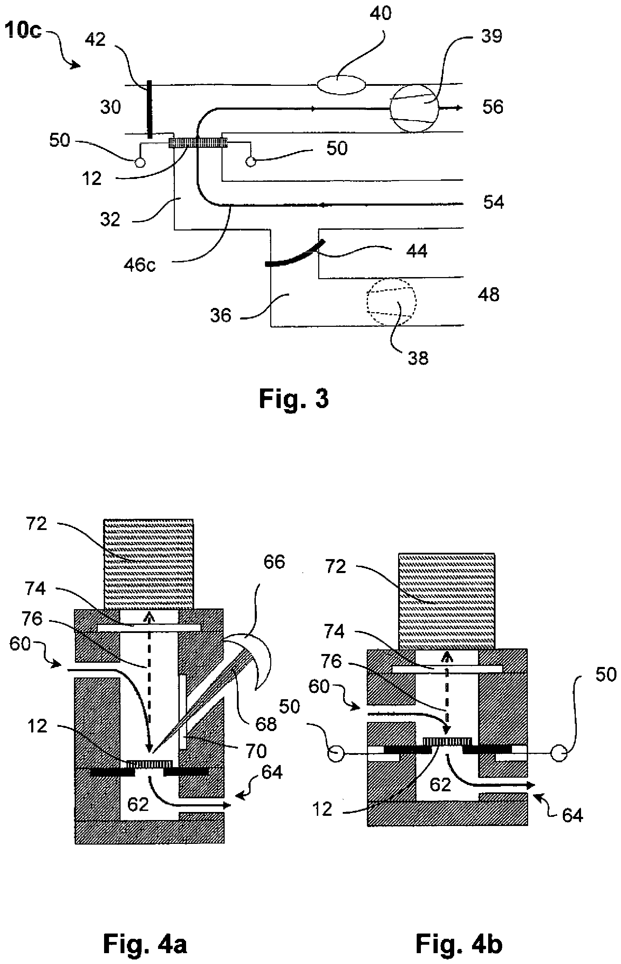

In **Figur 3** ist eine weitere Ausführungsform **10c** für eine Detektionsvorrichtung dargestellt, die im Wesentlichen dem Vergleichsbeispiel gemäß **10b** aus den Figuren 2a und 2b entspricht. Im Unterschied dazu ist kein geschlossener Zirkulationskanal vorhanden, sondern vielmehr ist der Strömungskanal **32** mit einem Einlass **54** und einem Auslass **56** verbunden. Bei dieser Ausführungsform wird im Detektionsmodus das Gas nicht zirkuliert, sondern über den Einlass 54 angesaugt, durch den Mikrofilter **12** geleitet und über die Saugpumpe 39. zum Auslass 56 geführt, wobei die vom Gasstrom **46c** mitgenommenen Sprengstoffpartikel wiederum vom Detektor **40** detektiert werden. Dabei wird der Mikrofilter **12** wiederum über die Anschlüsse **50** elektrisch beheizt.

## Patentansprüche

1. Verfahren zur Detektion von Sprengstoffpartikeln in einem Gasstrom (46), bei dem der Gasstrom (46) für einen vorgegebenen Zeitraum durch ein Adsorptionsnetz (12) geleitet wird, wobei Sprengstoffpartikel (18) darauf adsorbiert werden, anschließend das Adsorptionsnetz (12) auf eine Heiztemperatur erwärmt wird, bei der die Sprengstoffpartikel (18) desorbieren und ein Gasstrom mit den desorbierten Sprengstoffpartikeln einem Detektor (40) zu deren Detektion zugeführt wird, wobei als Adsorptionsnetz ein Mikrofilter (12) mit einer Porengröße kleiner als der Partikelgröße der zu detektierenden Sprengstoffpartikel (18) verwendet wird, **dadurch gekennzeichnet, dass** eine Heiztemperatur eingestellt und ein Mikrofilter (12) mit einer Porengröße verwendet wird und dass die zu detektierenden Sprengstoffpartikel (18), nach dem Erwärmen und Desorbieren in der Gasphase, den Mikrofilter (12) durchströmen.

2. Verfahren nach Anspruch 1, **dadurch gekennzeichnet, dass** ein Mikrofilter (12) mit einer Porengröße von kleiner 1µm, vorzugsweise von kleiner 400 nm, verwendet wird.

3. Verfahren nach einem der vorherigen Ansprüche, **dadurch gekennzeichnet, dass** der Mikrofilter (12) auf eine bestimmte Temperatur zur Detektion bestimmter Sprengstoffe erwärmt wird.

4. Vorrichtung zur Durchführung des Verfahrens nach Anspruch 1, **dadurch gekennzeichnet, dass** diese Vorrichtung als Adsorptionsnetz einen Mikrofilter (12) mit einer Porengröße kleiner als der Partikelgröße der zu detektierenden Sprengstoffpartikel umfasst, hinter dem ein Gasdetektor (40) zur Detektion von desorbierten Sprengstoffpartikeln angeordnet ist, wobei der Mikrofilter (12) eine Heizvorrichtung (50, 66) sowie eine Regelvorrichtung zur Regelung der Temperatur des Mikrofilters (12) umfasst, wobei der Gasstrom (46) den Mikrofilter (12) permanent durchströmt und der Gasdetektor (40) ständig von dem Gasstrom (46) überströmt wird.

5. Vorrichtung nach Anspruch 4, **dadurch gekennzeichnet, dass** diese einen Halogenstrahler (66) zur Erwärmung des Mikrofilters (12) sowie einen Temperatursensor (72) zur Erfassung der Temperatur des Mikrofilters (12) umfasst.

6. Vorrichtung nach Anspruch 5, **dadurch gekennzeichnet, dass** der Mikrofilter (12) resistiv beheizbar ist und ein Temperatursensor (72) zur Erfassung der Temperatur des Mikrofilters (12) vorgesehen ist.

## Claims

1. Method for detecting explosive-substance particles in a gas flow (46), in which the gas flow (46) is guided for a predetermined period of time through an adsorbent bed (12), wherein explosive-substance particles (18) are adsorbed thereupon, subsequently the adsorbent bed (12) is heated to a heating temperature at which the explosive-substance particles (18) desorb and a gas flow with the desorbed explosive-substance particles is conveyed to a detector (40) for their detection, wherein as adsorbent bed is used a microfilter (12) with a pore size smaller than the particle size of the explosive-substance particles (18) to be detected, **characterised in that** a heating temperature is set and a microfilter (12) with a pore size is used and that the explosive-substance particles (18) to be detected, after the heating and desorbing in the gaseous phase, flow through the microfilter (12).

2. Method according to claim 1, **characterised in that** a microfilter (12) is used having a pore size of less than 1 µm, preferably of less than 400 nm.

3. Method according to any of the preceding claims, **characterised in that** the microfilter (12) is heated to a specified temperature for the detection of specified explosive substances.

4. Device for carrying out the method according to claim 1, **characterised in that** this device as adsorbent bed comprises a microfilter (12) having a pore size less than the particle size of the explosive-substance particles to be detected, behind which a gas detector (40) is disposed for the detection of desorbed explosive-substance particles, wherein the microfilter (12) comprises a heating device (50, 66) and a regulating device for the regulation of the temperature of the microfilter (12), wherein the gas flow (46) flows permanently through the microfilter (12) and the gas flow (46) flows continuously over the gas detector (40).

5. Device according to claim 4, **characterised in that** it comprises a halogen lamp (66) for the heating of the microfilter (12) and a temperature sensor (72) for the detection of the temperature of the microfilter (12).

6. Device according to claim 5, **characterised in that** the microfilter (12) can be heated resistively and a temperature sensor (72) is provided for the detection of the temperature of the microfilter (12).

## Revendications

1. Procédé de détection de particules d'explosif dans un flux gazeux (46), dans lequel le flux gazeux (46) est dirigé pendant un laps de temps prédéfini à travers un maillage d'adsorption (12), dans lequel des particules d'explosif (18) sont adsorbées sur celui-ci, ensuite le maillage d'adsorption (12) est chauffé à une température de chauffage, à laquelle les particules d'explosif (18) se désorbent et un flux gazeux avec les particules d'explosif désorbées est amené à un détecteur (40) pour leur détection, dans lequel un microfiltre (12) avec une taille de pore inférieure à la taille de particule des particules d'explosif (18) à détecter est utilisé en tant que maillage d'adsorption, **caractérisé en ce qu'**une température de chauffage est réglée et un microfiltre (12) avec une taille de pore est utilisé et que les particules d'explosif (18) à détecter traversent le microfiltre (12), après le chauffage et la désorption dans la phase gazeuse.

2. Procédé selon la revendication 1, **caractérisé en ce qu'**un microfiltre (12) avec une taille de pore inférieure à 1 µm, de préférence inférieure à 400 nm, est utilisé.

3. Procédé selon l'une quelconque des revendications précédentes, **caractérisé en ce que** le microfiltre (12) est chauffé à une température déterminée pour la détection d'explosifs déterminés.

4. Dispositif pour l'exécution du procédé selon la revendication 1, **caractérisé en ce que** ce dispositif comprend, en tant que maillage d'adsorption, un microfiltre (12) avec une taille de pore inférieure à la taille de particule des particules d'explosif à détecter, derrière lequel un détecteur de gaz (40) pour la détection de particules d'explosif désorbées est agencé, dans lequel le micro filtre (12) comprend un dispositif de chauffage (50, 66) ainsi qu'un dispositif de réglage pour le réglage de la température du microfiltre (12), dans lequel le flux gazeux (46) traverse en permanence le microfiltre (12) et le détecteur de gaz (40) est constamment soumis à l'écoulement du flux gazeux (46).

5. Dispositif- selon la revendication 4, **caractérisé en ce que** celui-ci comprend une lampe halogène (66) pour le chauffage du microfiltre (12) ainsi qu'un capteur de température (72) pour la détection de la température du microfiltre (12).

6. Dispositif selon la revendication 5, **caractérisé en ce que** le microfiltre (12) peut être chauffé de manière résistive et un capteur de température (72) est prévu pour la détection de la température du microfiltre (12).
